## Europäisches Patentamt
## European Patent Office
## Office européen des brevets

(11) Veröffentlichungsnummer: **0 272 380 B1**

# EUROPÄISCHE PATENTSCHRIFT

(45) Veröffentlichungstag der Patentschrift: **08.07.92**

(21) Anmeldenummer: **87113004.3**

(22) Anmeldetag: **05.09.87**

(51) Int. Cl.5: **A61K 7/16**, **C01B 33/154**, **C09C 1/30**

(54) **Fällungskieselsäuren, Verfahren zu ihrer Herstellung und Verwendung.**

(30) Priorität: **21.11.86 DE 3639845**

(43) Veröffentlichungstag der Anmeldung:
**29.06.88 Patentblatt 88/26**

(45) Bekanntmachung des Hinweises auf die
Patenterteilung:
**08.07.92 Patentblatt 92/28**

(84) Benannte Vertragsstaaten:
**AT BE CH DE ES FR GB LI NL SE**

(56) Entgegenhaltungen:
EP-A- 0 062 749          EP-B- 0 018 866
EP-B- 0 078 909          DE-A- 1 467 019
DE-C- 1 293 138          US-A- 4 140 757

(73) Patentinhaber: **Degussa Aktiengesellschaft**
**Weissfrauenstrasse 9**
**W-6000 Frankturt am Main 1(DE)**

(72) Erfinder: **Reinhardt, Helmut, Dr.**
**Auf dem Stumpelrott 9**
**W-5000 Köln(DE)**
Erfinder: **Becker, Adam**
**Weserstr. 17**
**W-5303 Bornh.-Hersel(DE)**
Erfinder: **Kuhlmann, Robert**
**Paul Keller-Str. 24**
**W-5042 Erftstadt(DE)**
Erfinder: **Nauroth, Peter**
**Germanusstr. 8**
**W-5047 Wesseling(DE)**

**Beschreibung**

Die Erfindung betrifft Fällungskieselsäuren, Verfahren zu ihrer Herstellung und ihre Verwendung in Zahnpasten.

Synthetisch hergestellte Fällungskieselsäuren spielen seit vielen Jahren als Bestandteil von Zahnpflegemitteln eine große Rolle. Sie sind sehr rein, toxikologisch unbedenklich und mit den anderen Inhaltsstoffen von Zahnpasten, wie z.B. Glyzerin, Sorbitol, Verdickungsmitteln, Detergentien, Farb- und Aromastoffen und, gegebenenfalls, löslichen Fluorverbindungen, verträglich.

Synthetische Fällungskieselsäuren werden durch Fällung aus Alkalisilikatlösungen mit Säuren in einem Rührprozeß, Abfiltrieren der Suspension, Auswaschen, Trocknen und Mahlen hergestellt. Der Fällprozeß bietet die Möglichkeit, bereits durch Eingriff in die Flöckchenphase wichtige Eigenschaften wie Korndurchmesser, Kornform und Korndichte bzw. -Härte vorherzubestimmen.

Gegenstand der Erfindung ist ein Verfahren zur Herstellung von reinigenden, polierenden und verdikkenden Fällungskieselsäuren für Zahnpflegemittel, welches dadurch gekennzeichnet ist, daß man die in üblicher Weise auf dem Fällungswege erhaltenen Kieselsäuren unterschiedlicher Teilchengröße und Teilchendichte in der Suspensionsphase homogen miteinander vermischt und die Gemische in üblicher Weise durch Filtrieren, Waschen, Trocknen und Vermahlen aufarbeitet.

Ein weiterer Gegenstand der Erfindung ist eine Fällungskieselsäure, die durch die folgenden physikalisch-chemischen Kenndaten gekennzeichnet ist:

| | | | |
|---|---|---|---|
| Trocknungsverlust (DIN 53 198) | % | 3 | – 7 |
| Leitfähigkeit bei 25°C (4 %ige Aufschlämmung) | /uS | 400 | – 800 |
| pH-Wert ( 5 % nach DIN 53 200) | | 6 | – 7 |
| BET-Oberfläche (DIN 66 131) | $m^2$/g | 130 | – 150 |
| Stampfdichte (DIN 53 194) | g/l | 100 | – 150 |
| Makroporenvolumen D >30 nm (nach der Hg-Einpreßmethode) | ml/g | 3,2 | – 4,0 |
| Ölaufnahme n. Gardner | ml/100 g | 180 | – 200 |
| Wasserrückhaltevermögen | % | 76 | – 79 |
| Cu-Abrieb | mg | 5 | – 14 |
| RDA-Abrieb | | 35 | – 100 |
| REA-Abrieb | | 40 | – 90 |
| Kratzer | | wenige | – sehr wenige |
| Viskosität (in 16 %iger Glyzerin. Wasser-Dispersion 1:1 Spindel D mit Helipath) | mPas | 5000 | – 10000 |
| Fe-Gehalt | ppm | 240 | – 280 |

Ein weiterer Gegenstand der Erfindung ist ein Verfahren zur Herstellung der Fällungskieselsäure, die durch die folgenden physikalisch-chemischen Kenndaten gekennzeichnet ist:

| | | | | |
|---|---|---|---|---|
| Trocknungsverlust (DIN 53 198) | % | 3 | – | 7 |
| Leitfähigkeit bei 25°C ( 4 %ige Aufschlämmung) | /uS | 400 | – | 800 |
| pH-Wert ( 5 % nach DIN 53 200) | | 6 | – | 7 |
| BET-Oberfläche (DIN 66 131) | $m^2/g$ | 130 | – | 150 |
| Stampfdichte (DIN 53 194) | g/l | 100 | – | 150 |
| Makroporenvolumen D >30 nm (nach der Hg-Einpreßmethode) | ml/g | 3,2 | – | 4,0 |
| Ölaufnahme n. Gardner | ml/100 g | 180 | – | 200 |
| Wasserrückhaltevermögen | % | 76 | – | 79 |
| Cu-Abrieb | mg | 5 | – | 14 |
| RDA-Abrieb | | 35 | – | 100 |
| REA-Abrieb | | 40 | – | 90 |
| Kratzer | | wenige | – | sehr wenige |
| Viskosität (in 16 %iger Glyzerin-Wasser-Dispersion 1:1 Spindel D mit Helipath) | mPas | 5000 | – | 10000 |
| Fe-Gehalt | ppm | 240 | – | 280 |

welches dadurch gekennzeichnet ist, daß man zum einen eine Verdickungsfällungskieselsäuresuspenion durch Umsetzung einer Alkalisilikatlösung mit einer Säure unter Vermeidung einer Gelbildung herstellt, wobei die Alkalisilikatlösung und die Säure gleichzeitig in eine vorgelegte wässrige Alkalisilikatlösung eingespeist werden, wobei zur Fällung der Kieselsäure in eine vorgelegte Alkalisilikatlösung mit einer Konzentration von etwa 5 bis 25 g $SiO_2$ je Liter Lösung, die Säure und die Alkalisilikatlösung mit bestimmten Lösungskonzentrationen und bestimmten Zulaufgeschwindigkeiten unter Aufrechterhaltung einer Fälltemperatur im Reaktionsmedium zwischen 80 und 90°C derartig eingespeist werden, daß die Viskosität des Reaktionsmediums in einem Zeitraum von mindestens 30 % der gesamten Fälldauer gleichmäßig niedrig und der pH-Wert zwischen 10 und 12 gehalten wird, und die Zugabe der Reaktionsteilnehmer beendet wird, bevor die Viskosität nach Durchlaufen eines Maximums auf einen weniger als 100 % über der Anfangsviskosität liegenden Wert gesunken ist, den pH-Wert mit Schwefelsäure unter 7 eingestellt wird, zum anderen eine Abrasivfällungskieselsäuresuspension herstellt, indem man eine Originalfällungskieselsäuresuspension, die durch Fällung der Kieselsäure aus einer vorgelegten Alkalisilikatlösung mit einer Konzentration von etwa 5 - 25 g $SiO_2$ je Liter Lösung mit einer Säure und Alkalimetallsilikatlösung mit bestimmten Lösungskonzentrationen und bestimmten Zulaufgeschwindigkeiten unter Aufrechterhaltung einer Fälltemperatur im Reaktionsmedium zwischen 80 und 90°C, wobei gegebenfalls der Behälterinhalt während der gesamten Fälldauer intensiv geschert wird, derart hergestellt wird, daß die Viskosität des Reaktionsmediums in einem Zeitraum von mindestens 30 % der gesamten Fälldauer gleichmäßig niedrig und der pH-Wert zwischen 10 und 12 gehalten wird, und die Zugabe der Reaktionsteilnehmer beendet ist, bevor die Viskosität nach Durchlaufen eines Maximums auf einen weniger als 100 % über der Anfangsviskosität liegenden Wert gesunken ist, den pH-Wert mit Schwefelsäure auf 7 bis 9 einstellt, mit heißem Wasser auf einen Fällungskieselsäuregehalt von 10 - 30 g/l und Natriumsulfatgehalt von 6 - 20 g $Na_2SO_4$/l verdünnt, auf 80 - 90 °C erwärmt, und unter Konstanthalten dieses pH-Wertes durch gleichzeitigen Zulauf von Alkalimetallsilikatlösung, Schwefelsäure und gegebenenfalls heißem Wasser über eine Fälldauer von 15 bis 180 Minuten eine Fällungskieselsäureendkonzentration von 40 bis 80 g/l einstellt, die Suspension mit Schwefelsäure auf einen pH-Wert unter 7 ansäuert, beide Fällungskieselsäuresuspensionen miteinander vermischt, die Fällungskieselsäure abfiltriert, wäscht, sprühtrocknet und anschliessend mit der Luftstrahlmühle vermahlt. In einer bevorzugten Ausführungsform der Erfindung wird die Verdickungskieselsäuresuspension im wesentlichen gemäß DE-AS 14 67 019 hergestellt. Die Abrasivfällungskieselsäuresuspension wird vorzugsweise im wesentlichen gemäß DE-OS 31 14 493, Beispiel 10 hergestellt.

Ein weiterer Gegenstand der Erfindung ist eine Fällungskieselsäure, gekennzeichnet durch die folgen-

den physikalisch-chemischen Kenndaten:

| | | | | |
|---|---|---|---|---|
| Trocknungsverlust (DIN 53 198) | % | 3 | – | 7 |
| Leitfähigkeit bei 25°C ( 4 %ige Aufschlämmung) | /uS | 250 | – | 400 |
| pH-Wert ( 5 % nach DIN 53 200) | | 6 | – | 7 |
| BET-Oberfläche (DIN 66 131) | $m^2/g$ | 220 | – | 250 |
| Stampfdichte (DIN 53 194) | g/l | 80 | – | 120 |
| Makroporenvolumen D > 30 nm (nach der Hg-Einpreßmethode) | ml/g | 3,2 | – | 4,0 |
| Ölaufnahme n. Gardner | ml/100 g | 200 | – | 220 |
| Wasserrückhaltevermögen | % | 77 | – | 78 |
| Cu-Abrieb | mg | 8 | – | 12 |
| RDA-Abrieb | | 60 | – | 80 |
| REA-Abrieb | | 60 | – | 90 |
| Kratzer | | wenige | | |
| Viskosität (in 16 %iger Glyzerin-Wasser-Dispersion 1:1 Spindel D mit Helipath) | mPas | 14000 | – | 18000 |
| Fe-Gehalt | ppm | 70 | – | 90 |

Ein weiterer Gegenstand der Erfindung ist ein Verfahren zur Herstellung der Fällungskieselsäure, die die folgenden physikalisch-chemischen Kenndaten aufweist:

| | | |
|---|---|---|
| Trocknungsverlust (DIN 53 198) | % | 3 - 7 |
| Leitfähigkeit bei 25°C (4 %ige Aufschlämmung) | /uS | 250 - 400 |
| pH-Wert ( 5 % nach DIN 53 200) | | 6 - 7 |
| BET-Oberfläche (DIN 66 131) | $m^2/g$ | 220 - 250 |
| Stampfdichte (DIN 53 194) | g/l | 80 - 120 |
| Makroporenvolumen D > 30 nm (nach der Hg-Einpreßmethode) | ml/g | 3,2 - 4,0 |
| Ölaufnahme n. Gardner | ml/100g | 200 - 220 |
| Wasserrückhaltevermögen | % | 77 - 78 |
| Cu-Abrieb | mg | 8 - 12 |
| RDA-Abrieb | | 60 - 80 |
| REA-Abrieb | | 60 - 90 |
| Kratzer | | wenige |
| Viskosität (in 16 %iger Glyzerin-Wasser-Dispersion 1:1 Spindel D mit Helipath) | mPas | 14000- 18000 |
| Fe-Gehalt | ppm | 70 - 90 |

welches dadurch gekennzeichnet ist, daß man zum einen eine Abrasivfällungskieselsäuresuspension herstellt, indem man eine Originalfällungskieselsäuresuspension, die durch Fällung der Kieselsäure aus einer vorgelegten Alkalisilikatlösung mit einer Konzentration von etwa 5 - 25 g $SiO_2$ je Liter Lösung mit einer Säure und Alkalimetallsilikatlösung mit bestimmten Lösungskonzentrationen und bestimmten Zulaufgeschwindigkeiten unter Aufrechterhaltung einer Fälltemperatur im Reaktionsmedium zwischen 80 und 90°C derart hergestellt wird, daß die Viskosität des Reaktionsmediums in einem Zeitraum von mindestens 30 % der gesamten Fälldauer gleichmäßig niedrig und der pH-Wert zwischen 10 und 12 gehalten wird, und die Zugabe der Reaktionsteilnehmer beendet wird, bevor die Viskosität nach Durchlaufen eines Maximums auf einen weniger als 100 % über der Anfangsviskosität liegenden Wert gesunken ist, den pH-Wert mit Schwefelsäure auf 7 bis 9 einstellt, mit heißem Wasser auf einen Fällungskieselsäuregehalt von 10 - 30 g/l und Natriumsulfatgehalt von 6 - 20 g $Na_2SO_4$/l verdünnt, auf 80 - 90°C erwärmt, den pH-Wert mit Schwefelsäure auf 7 bis 9 einstellt und unter Konstanthalten dieses pH-Wertes durch gleichzeitigen Zulauf von Alkalimetallsilikatlösung, Schwefelsäure und gegebenenfalls heißem Wasser über eine Fälldauer von 15 bis 180 Minuten eine Fällungskieselsäureendkonzentration von 40 bis 80 g/l einstellt, die Suspension mit Schwefelsäure auf einen pH-Wert unter 7 ansäuert,
zum anderen eine weitere Fällungskieselsäuresuspension herstellt, indem man die Fällung im stark sauren pH-Bereich, vorzugsweise bei einem pH-Wert zwischen 1,5 und 2,5 durch an sich bekannte gleichzeitige Zugabe der Wasserglas- und Säurelösungen zu einer Vorlage von in Wasser gelösten Stoffen, welche mit Eisen lösliche Komplexverbindungen eingehen, durchführt, beide Fällungskieselsäuresuspensionen miteinander vermischt, die Fällungskieselsäure abfiltriert, wäscht, sprühtrocknet und anschließend mit der Luftstrahlmühle vermahlt. In einer bevorzugten Ausführungsform wird die Abrasivfällungskieselsäuresuspension im wesentlichen gemäß DE-OS 31 14 493 , Beispiel 10, hergestellt. Die weitere Fällungskieselsäuresuspension wird vorzugsweise gemäß DE-PS 12 93 138 hergestellt, wobei als Komplexbildner die dort angegebenen Substanzen, vorzugsweise Natriumfluorid und Oxalsäure, eingesetzt werden.

Es wurde nun gefunden, daß man durch Mischen von Fällungskieselsäuren unterschiedlicher Eigenschaften in der Suspensionsphase zu besonders wertvollen Fällungskieselsäuren für den Einsatz in Zahnpflegemitteln kommt. Geeignete Fällungskieselsäuresuspensionen sind direkt durch Fällung oder durch Wiederaufschlämmung von Fällungskieselsäurefilterpressenteig erhältlich. Vorzugsweise werden direkt durch Fällung hergestellte Fällungskieselsäuresuspensionen verwendet.

Die durch das erfindungsgemäße Mischen von Fällungskieselsäuresuspensionen durch Filtrieren, Trocknen und Vermahlen gewonnenen Fällungskieselsäuren haben in Zahnpflegemitteln günstigere Eigen-

EP 0 272 380 B1

schaften als Trockenmischungen derselben Ausgangsfällungskieselsäuren im gleichen Verhältnis.

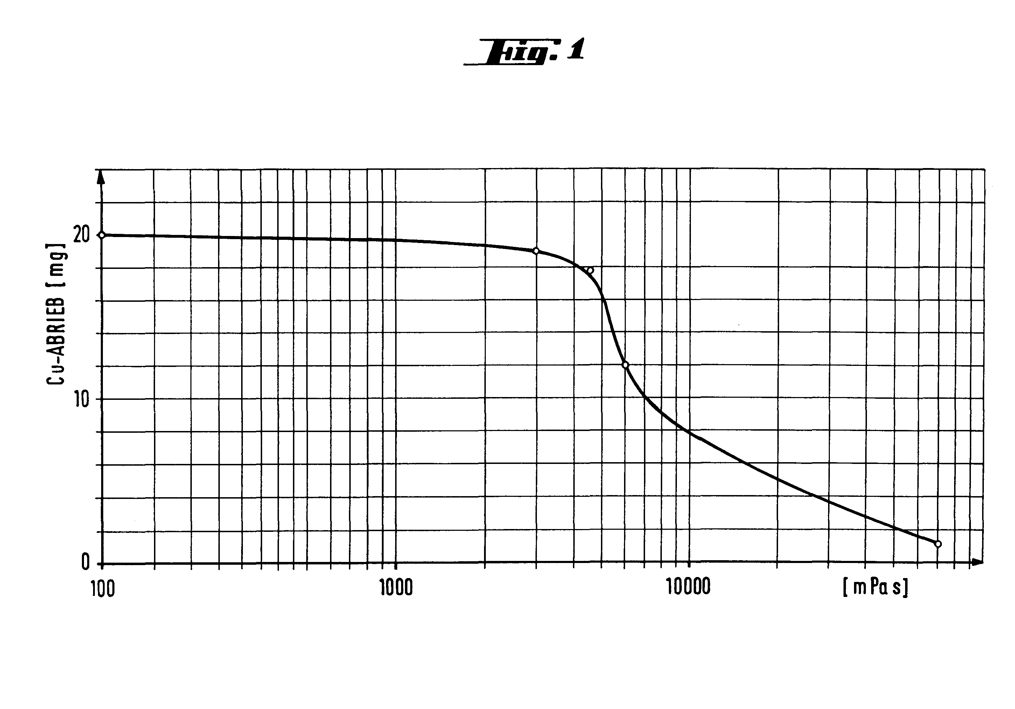

Die erfindungsgemässen Fällungskieselsäuren zeigen synergistische Effekte derart, daß bei gegebener Viskositäts- bzw. Verdickungswirkung eine unerwartet hohe Abrasivität auftritt, d.h., die Viskositäts- Abrasivitätsbeziehung verläuft überraschenderweise nicht linear (vgl. Figur 1).

Die erfindungsgemässen Fällungskieselsäuren lassen sich vorteilhafterweise besonders leicht herstellen, weil die Mengen über geeignete Volumen- oder Gewichtsmessungen bei bekannten Feststoffgehalten kontinuierlich oder chargenweise exakt eingestellt werden können. Ferner wirft der Naßprozess vorteilhafterweise keine Staubprobleme auf. Auch Entmischungserscheinungen sind minimiert.

Anscheinend verlieren in Naßmischungen im Unterschied zu Trockenmischungen die Komponenten einen Teil ihrer Identität. Beim Vermischen und vermutlich besonders beim Trocknen kommt es zu Wechselwirkungen kleiner mit größeren Teilchen, wobei die kleinen Teilchen an größere angelagert werden oder aufwachsen.

Auch verschiedene Formen gegenseitiger Flockung und Durchdringung in der wässrigen Phase sind in Betracht zu ziehen.

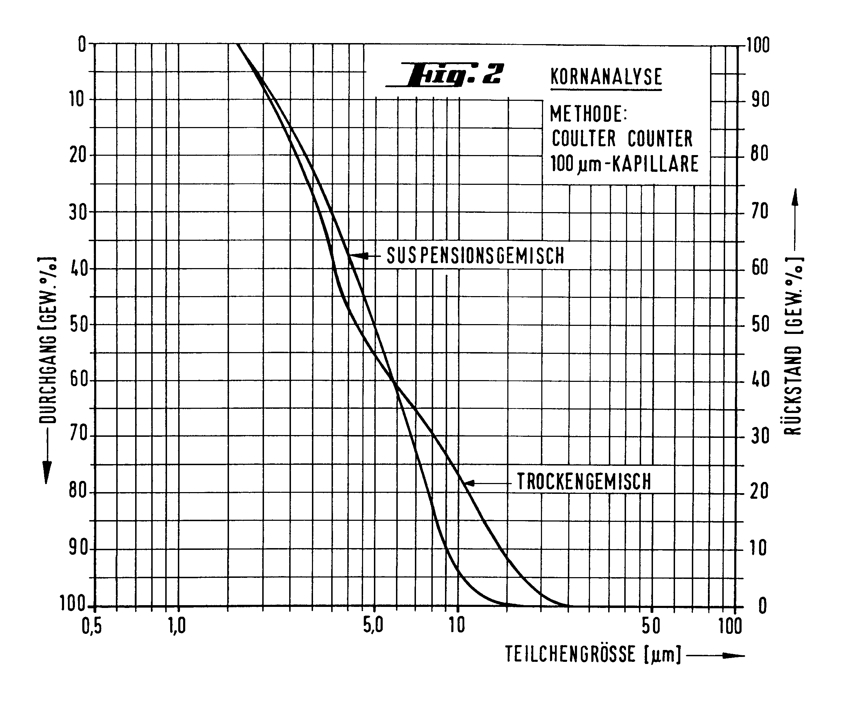

Eine erste deutliche Unterscheidung zwischen Trocken- und Naßmischung ergibt sich aus der Teilchengrößenverteilung der gemahlenen Proben. Während die Trockenmischung zweier Fällungskieselsäuren mit unterschiedlicher Korngröße und Dichte bei der Vermessung im Coulter Counter erwartungsgemäß 2 Maxima zeigt und die entsprechende Kurve einen Höcker aufweist, verläuft die Kurve der gleichen Fällungskieselsäuren, im gleichen Verhältnis naß miteinander gemischt, vollkommen harmonisch (vgl. Figur 2).

Ein weiterer merklicher Unterschied besteht in der Verdickungswirkung. Die erfindungsgemäße Fällungskieselsäure erhöht die Viskosität einer Modellmischung aus Glyzerin und Wasser deutlich weniger als das Trockengemisch.

Fällungskieselsäuren mit mäßiger Verdickungskraft erlauben höhere Füllungsgrade und damit die Herstellung von Pasten mit "vollem" Charakter.

Niedrig gefüllte Pasten wirken demgegenüber im Mund "leer" bzw. "wässrig".

Die verdickende Wirkung der Fällungskieselsäure muß sich bei der Einarbeitung in die Zahnpasta-Masse voll entfalten, d.h. es darf danach keine Veränderung des rheologischen Verhaltens der Zahnpasta auftreten, die das Befüllen und spätere Entleeren der Tuben und Behältnisse erschwert oder gar unmöglich macht.

Grundsätzlich soll ein Zahnpflegemittel die Reinigungswirkung der Zahnbürste unterstützen bzw. erhöhen, indem zwischen Borste und Zahnoberfläche ein Film aufgebaut wird, der Zahnbeläge mechanisch entfernt.

Diese Leistung kann nur von einem Mittel mit abrasiven Eigenschaften erbracht werden.

Bei gleicher REA-Abriebleistung sind selbstverständlich Fällungskieselsäuren zu bevorzugen, die die wenigsten Kratzer erzeugen.

Die erfindungsgemässen Fällungskieselsäuren vereinigen in vorteilhafter Weise Forderungen nach stabiler Viskositätsentwicklung in mittlerer Höhe mit hoher Abrasiv- und Reinigungswirkung unter Vermeidung tiefer Kratzer.

Die Messung der Abrasivität wird bei extrahierten, radioaktiv gemachten menschlichen Zähnen an freigelegtem Zahnbein (RDA-Wert) oder an Zahnschmelz (REA-Wert) durchgeführt. Gemessen wird jeweils die Zunahme der Radioaktivität der Suspension des zu prüfenden Zahnpflegemittels nach definierter Bearbeitung der Probezähne mit Bürsten.

Ersatzweise kann die Gewichtsabnahme von Kupferplatten beim Bürsten mit Prüfsuspension bestimmt werden. Die so erhaltenen Werte lassen sich aber häufig nicht mit RDA- und REA-Werten vergleichen.

In Speziallaboratorien erhält man mit verfeinerten, z.B. mikroskopischen, Methoden Aufschluß über die reinigende und polierende Wirkung von Zahnpflegemitteln, wobei Proben, die viele oder tiefe Kratzer verursachen, ausgeschieden werden. Diese Untersuchungen werden an extrahierten tierischen oder menschlichen Zähnen durchgeführt.

Einen Eindruck von Größe und Anzahl der von einem Mittel erzeugten Kratzer gewinnt man schon bei mikroskopischer oder stereomikroskopischer Betrachtung definiert behandelter Metallplatten, wie sie beispielsweise bei der Messung des Kupferabriebs anfallen.

Neben Verdickungseigenschaften und schonender Reinigungs- und Polierwirkung werden heute von einer Fällungskieselsäure aus gesundheitlichen Gründen hohe Reinheit in Bezug auf Schwermetallgehalte erwartet. Höhere Eisengehalte können darüberhinaus zu Verfärbungen der Pasten, die teilweise farbig transparent eingestellt sind, führen. So kann eine mit Indigotin blau angefärbte Paste durch eisenhaltige Kieselsäuren einen grünlichen Mißton erhalten.

Wir haben nun weiter gefunden, daß man auf dem Wege der Naßmischung bei Auswahl gewisser

6

Kieselsäuren zu sehr reinen Produkten mit besonders niedrigen Eisengehalten kommt.

Ein weiterer Gegenstand der Erfindung ist die Verwendung der erfindungsgemäßen Fällungskieselsäure als Abrasiv- bzw. Poliermittel in Zahnpasten.

Beispiel

Der Feststoffgehalt (Fällungskieselsäuregehalt) der Fällungskieselsäuresuspensionwird wie folgt bestimmt:

250 ml der auf 20°C abgekühlten Fällungskieselsäuresuspension werden auf einer Porzellannutsche (⌀ 120 mm) abgenutscht und danach der Filterkuchen mit 500 ml Wasser elektrolytarm gewaschen. Der Kuchen wird bei 105°C bis zur Gewichtskonstanz getrocknet.

Berechnung:     g Auswaage $\times$ 4 = g Feststoff/l

Beispiel 1

Es wird eine Fällungskieselsäure mit Verdickungswirkung im wesentlichen gemäß DE-AS 14 67 019 hergestellt.

In Abänderung hierzu wird aus ökonomischen Gründen konzentrierte Natriumsilikatlösung (Dichte $\rho$ = 1,353 g/ml) und konzentrierte Schwefelsäure (96 %) eingesetzt. Hieraus resultiert ein Feststoffgehalt von 85 g/l in der Fällungskieselsäuresuspension.

In einem gummierten 120 l-Fällgefäß werden 73 l heisses Wasser und 5,25 l Natriumsilikatlösung (Dichte $\rho$ = 1,353 g/ml, Modul $SiO_2$ $Na_2O$ = 3,46) unter Rühren auf 85°C erhitzt. In diese alkalische Fällungsvorlage werden während der folgenden 90 Minuten unter Rühren und Aufrechterhaltung der Temperatur von 85°C gleichzeitig 16,5l Natriumsilikatlösung (Dichte $\rho$ = 1,353 g/ml, Modul $SiO_2$ $Na_2O$ = 3,46) mit 11,0 l/Stunde und 1,448 l Schwefelsäure (96 %ig) mit 0,965 l/Stunde dosiert. Die Zugabe der Reaktionsteilnehmen wird beendet, bevor die Viskosität des Reaktionsmediums nach Durchlaufen eines Maximums auf einen weniger als 100 % über der Anfangsviskosität liegenden Wert gesunken ist.

Danach wird die so erhaltene Fällungskieselsäuresuspension mit Schwefelsäure (96 %ig) auf einen pH-Wert von 3,5 eingestellt, was durch einen mehrere Minuten lang andauernden Säurezufluß mit 1,25 l/Stunde geschieht. Die auf diese Weise erhaltene Fällungskieselsäuresuspension hat einen Feststoffgehalt von 85,0 g/l .

Die nach dem Abfiltrieren, Auswaschen, Sprühtrocknen und Vermahlen auf der Luftstrahlmühle erhaltene Fällungskieselsäure hat die in der Tabelle 1 aufgeführten physikalisch-chemischen Kenndaten.

Beispiel 2

Das Beispiel beschreibt die Herstellung einer Abrasivfällungskieselsäure gemäß DE-OS 31 14 493, Beispiel 10, jedoch mit der Abänderung, daß die gesamte Wassermenge zur Fällungsvorlage gegeben wird, wodurch die Wasserzugabe während der Fällung entfällt, und die Fällzeit von 60 auf 100 Minuten verlängert wird.

In einem gummierten 120 l-Fällgefäß werden 73 l heißes Wasser und 5,25 l Natriumsilikatlösung (Dichte = 1,353 g/ml; Modul: $SiO_2$ :$Na_2O$ = 3,46) unter Rühren auf 85°C erhitzt. In diese alkalische Fällungsvorlage werden während der folgenden 90 Minuten unter Rühren und Aufrechterhaltung der Temperatur von 85°C gleichzeitig 16,5 l Natriumsilikatlösung (Dichte = 1,353 g/ml; Modul $SiO_2$:$Na_2O$ = 3,46) mit 11,o l/h und 1,448 l konzentrierte Schwefelsäure (96 %ig) mit 0,965 l/h dosiert. Die Zugabe der Reaktionsteilnehmer wird beendet, bevor die Viskosität des Reaktionsmediums nach Durchlaufen eines Maximums auf einen weniger als 100 % über der Anfangsviskosität liegenden Wert gesunken ist.

Danach wird die so erhaltene Fällungskieselsäuresuspension mit konzentrierter Schwefelsäure (96 %ig) auf einen pH-Wert von 8,5 eingestellt, was durch einen mehrere Minuten lang andauernden Säurezufluß mit 1,25 l/h geschieht. Die auf diese Weise erhaltene Fällungskieselsäuresuspension hat einen Feststoffgehalt von 85 g/l.Ihr $Na_2SO_4$-Gehalt liegt bei 55 g/l.

Während der gesamten Fälldauer von 90 Minuten wird mittels einer Kreiselpumpe, die den Behälterinhalt mehrfach umwälzt, intensiv geschert. Nähere Angaben über die apparative Einrichtung sowie über die Scherbedingungen finden sich in der DE-PS 17 67 332 und dort insbesondere in Spalte 8, Zeilen 31 - 68.

Die auf diese Weise hergestellte Originalfällungskieselsäuresuspension wird mit Wasser auf einen Fällungskieselsäuregehalt von 13 g/l und 8,5 g $Na_2SO_4$/l eingestellt.

16,06 l dieser Suspension werden in einem gummierten 120 l-Fällgefäß unter Rühren auf 85°C erhitzt. Unter Aufrechterhaltung dieser Temperatur und eines pH-Wertes von 8,5 werden für die Dauer von 100 Minuten gleichzeitig 23,1 l Wasserglaslösung (Dichte $\rho$ = 1,353 g/ml; Modul $SiO_2$ $Na_2O$ = 3,46) mit einer Geschwindigkeit von 231.0 ml/min und 1,94 l Schwefelsäure (96 %ig) mit einer Geschwindigkeit von 19,4 ml/min. zur Fällungskieselsäuresuspension hinzugegeben. Die Fällungskieselsäuresuspension wird anschliessend mit Schwefelsäure (96 %ig) auf einen pH-Wert von ca. 3,5 eingestellt. Die erhaltene Fällungskieselsäuresuspension hat einen Feststoffgehalt von 92,0 g/l.

Die nach dem Abfiltrieren und Auswaschen erhaltene Fällungskieselsäure hat die in der Tabelle 1 aufgeführten physikalisch-chemischen Kenndaten:

Beispiel 3

18.5 m³ Suspension der Abrasivfällungskieselsäuresuspension mit einem Fällungskieselsäuregehalt von 92,0 g/l gemäß Beispiel 2 werden mit 40,0 m³ Suspension einer Verdickungsfällungskieselsäure gemäß Beispiel 1 mit einem Fällungskieselsäuregehalt von 85,0 g/l entsprechend einem Gewichtsverhältnis von 1:2 - (bezogen auf den Fällungskieselsäuregehalt) gemischt.

Zur Weiterverarbeitung wird die Fällungskieselsäure dieses Suspensiongemisches mittels Filterpressen abgetrennt. Der Fällungskieselsäuregehalt im Filterpressteig beträgt 24 %. Der gewaschene und sprühgetrocknete Filterpressenteig wird luftstrahlvermahlen. Die erhaltene Fällungskieselsäure weist die in der Tabelle 1 aufgeführten physikalisch-chemischen Kenndaten auf.

Beispiel 4

1,0 kg der gewaschenen, getrockneten und vermahlenen Verdickungsfällungskieselsäuredes Beispiels 1 wird mit 2,0 kg der ebenfalls in vergleichbarer Weise gewaschenen, getrockneten und vermahlenen Abrasivfällungskieselsäure des Beispiels 2 trocken gemischt, d.h. das Gewichtsverhältnis der beiden Fällungskieselsäuren ist wie in Beispiel 3 1:2.

Die erhaltene Fällungskieselsäure weist die in der Tabelle 1 aufgeführten physikalisch-chemischen Kenndaten auf.

Beispiel 5

Nach Maßgabe der DE-OS 31 14 493.4, Beispiel 12, wird zunächst eine Abrasivfällungskieselsäure wie folgt hergestellt:

In einem mit einem Rührwerk ausgerüsteten Fällbottich werden 27,0 m³ heißes Wasser und 10,0 m³ Fällungskieselsäuresuspension im wesentlichen gemäß DE-AS 14 67 019, die nur bis pH 8,5 angesäuert wird, mit einem Fällungskieselsäuregehalt von 85 g/l vermischt und auf 85°C erhitzt. In Abänderung gegenüber der DE-AS 14 67 019 wird aus ökonomischen Gründen konzentrierte Natriumsilikatlösung (Dichte = 1,353 g/ml) und konzentrierte Schwefelsäure (96 %ig) eingesetzt. Hieraus resultiert ein Feststoffgehalt von 85 g/l in der Fällungskieselsäuresuspension. Bei dieser Temperatur sowie unter Aufrechterhaltung eines pH-Wertes von 8,5-9 werden für die Dauer von 80 Minuten gleichzeitig 9,6 m³ Natriumsilikatlösung (Dichte = 1,353 g/ml; Modul $SiO_2$ $Na_2O$ = 3,46), 0,94 m³ konzentrierte Schwefelsäure (96 %ig) und 20,0 m³ heißes Wasser hinzugegeben.

Nach beendeter Fällung wird die Suspension mit konzentrierter Schwefelsäure (96 %ig) auf einen pH-Wert von 3,5 gebracht. Der Fällungskieselsäuregehalt beträgt 66,0 g/l.

25,75 m³ dieser Fällungskieselsäuresuspension werden mit 40,0 m³ Suspension einer Verdickungskieselsäure gemäß Beispiel 1 mit einem Fällungskieselsäuregehalt von 85,0 g/l das entspricht einem Gewichtsverhältnis von 1 : 2, (bezogen auf den Feststoffgehalt) gemischt.

Zur Weiterverarbeitung wird wie im Beispiel 3 verfahren. Der Kieselsäuregehalt im Filterpressenteig beträgt 21 %.

Die erhaltene Fällungskieselsäure weist die in der Tabelle 1 aufgeführten physikalisch-chemischen Kenndaten auf.

Beispiel 6

Es wird eine eisenarme Fällungskieselsäure gemäß Beispiel 1 der D 47 633 (=DE-PS 12 93 138) hergestellt.

In einem zylindrischen, 30 l fassenden Holzbehälter, der mit einem hölzernen Blattrührer (Durchmesser 20 cm, Höhe 6 cm, 90 Umdrehungen pro Minute) versehen ist, werden 18,5 l Wasser vorgelegt.

Das Wasser wird mit einer Dampfschlange aus säurebeständigem Stahl indirekt auf 85°C erhitzt. In der Vorlage werden 25 g Natriumfluorid und 6,25 g Oxalsäure gelöst.

Nun werden unter Rühren und unter Einhalten eines pH-Wertes von 2,0 in 90 Minuten 5,45 l handelsübliches Natronwasserglas, (Molverhältnis $Na_2O : SiO_2 = 1:3,36$, Dichte $\rho = 1,34$) und 0,55 l Schwefelsäure (96 % $H_2SO_4$) an einander gegenüberliegenden Stellen eingetragen.

Die entstehende Suspension wird noch etwa 30 Minuten lang gerührt. Dann wird in bekannter Weise auf Nutschen abfiltriert, mit der zweifachen Menge Wasser, bezogen auf das Volumen der Suspension, ausgewaschen, sprühgetrocknet und luftstrahlvermahlen.

Beispiel 7

18,2 m³ Suspension der Abrasivkieselsäure gemäß Beispiel 2 mit einem Fällungskieselsäuregehalt von 92,0 g/l werden sodann mit 40,0 m³ Suspension einer eisenarmen Fällungskieselsäure gemäß Beispiel 1 der D 47 633 (=DE-PS 12 93 138) mit einem Fällungskieselsäuregehalt von 83,8 g/l, entsprechend einem Gewichtsverhältnis von 1:2, gemischt.

Zur Weiterverarbeitung wird wie in Beispiel 3 verfahren. Der Kieselsäuregehalt im Pressenteig beträgt 23 %.

Die erhaltene Fällungskieselsäure weist die in der Tabelle 1 aufgeführten physikalisch-chemischen Kenndaten auf.

Beispiel 8

Zur Herstellung einer eisenarmen Fällungskieselsäure mit verdickenden und reinigenden Eigenschaften wird die Suspension einer gemäß Beispiel 1 der DE-PS 12 93 138 (=D 47 633) hergestellten, eisenarmen Kieselsäure mit der Suspension der nach Beispiel 2 hergestellten abrasiven Kieselsäure in bestimmten Verhältnissen gemischt. Tabelle 2 zeigt, daß der Eisengehalt der Mischung bei jedem Gewichtsverhältnis niedriger liegt als der der vergleichbaren Trockenmischung.

## Tabelle 2

|  | Mischungsverhältnis in Gewichtsteilen | | |
|---|---|---|---|
| Abrasivkieselsäure, wie be-beschrieben | 2 | 1 | 1 |
| eisenarme Kieselsäure gemäß Beispiel 1 der D 47 633 | 1 | 1 | 2 |
| Naßmischung | 157 ppm Fe | 118 ppm Fe | 80 ppm Fe |
| Trockenmischung | 168 ppm Fe | 137 ppm Fe | 101 ppm Fe |

| | | Beisp. 1 | Beisp. 2 | Beisp. 3 | Beisp. 4 | Beisp. 5 | Beisp. 6 | Beisp. 7 |
|---|---|---|---|---|---|---|---|---|
| Trocknungsverlust (DIN 53 198) | % | 5 | 5 | 5 | 5 | 5 | 5 | 5 |
| Leitfähigkeit bei 25°C (4 %ige Aufschlämmung) | /uS | 730 | 460 | 740 | 650 | 680 | 250 | 290 |
| pH-Wert ( 5 % nach DIN 53 200) | | 6,3 | 6,9 | 6,3 | 6,4 | 6,3 | 6,1 | 6,5 |
| BET-Oberfläche (DIN 66 131) | m²/g | 185 | 40 | 140 | 145 | 148 | 310 | 235 |
| Stampfdichte (DIN 53 194) | g/l | 80 | 240 | 130 | 120 | 110 | 80 | 100 |
| Makroporenvolumen D >30 nm (nach der Hg-Einpreßmethode) | ml/g | 3,8 | 1,1 | 3,0 | 3,6 | 3,8 | 3,5 | 2,8 |
| Ölaufnahme n. Gardner | ml/100g | 280 | 80 | 184 | 215 | 195 | 300 | 200 |
| Wasserrückhaltevermögen | % | 80 | 56 | 76 | - | 79 | 84 | 81 |
| Cu-Abrieb | mg | 1 | 20 | 12 | 13 | 5 | 1 | 9 |
| RDA-Abrieb | | 23 | 170 | 80 | 100 | 35 | 15 | 70 |
| REA-Abrieb | | 25 | 130 | 70 | 40 | 45 | 20 | 75 |
| Kratzer | | sehr wenige | viel | wenige | viel | sehr wenige | sehr wenige | wenige |
| Viskosität (in 16 %iger Glyzerin-Wasser-Dispersion 1:1 Spindel D mit Helipath) | mPas | 72000 | <100 | 6000 | 10800 | 9500 | 170000 | 16500 |
| Fe-Gehalt | ppm | 260 | 240 | 240 | 260 | 250 | 35 | 80 |

Die physikalisch-chemischen Kenndaten, die in der Anmeldung angegeben werden, werden wie folgt bestimmt:

Trocknungsverlust nach DIN 53 198.

10

Leitfähigkeit bei 25°C
/ 4 %ige Aufschlämmung

Eine Probe von 4,0 g wird mit 50 ml vollentsalztem Wasser in einem 150 ml-Becherglas erhitzt und eine Minute unter Rühren gekocht. Anschließend wird die Suspension in einen 100 ml-Kolben überführt, abgekühlt und mit vollentsalztem Wasser bis zur Marke aufgefüllt. Die elektrische Leitfähigkeit wird mit einem handelsüblichen Meßgerät, z.B. der "Wissenschaftlich-Technischen Werkstätten" (WTW), Konduktometer Typ LF 530, bei 25°C gemessen.

pH-Wert in 5 % wässriger Dispersion nach DIN 53 200

BET-Oberfläche gemäß DIN 66 131

Eine Probe wird bei 100°C mit Stickstoff gespült. Die volumetrische Messung wird mit Reinststickstoff bei der Temperatur des flüssigen Stickstoffes (-195,8°C) durchgeführt.

Stampfdichte gemäß DIN 53 194

Die Untersuchung wird ohne Vorbehandlung der Probe vorgenommen.

Makroporenvolumen D >30 nm
nach der Hg-Einpreßmethode

Die Messung erfolgt mit dem Mercury Pressure Porosimeter, 200 Series, der Fa. Carlo Erba Strumentazione

Ölaufnahme n. Gardner-Coleman
gemäß ASTM : D 1483-60

Wasserrückhaltevermögen

Das Wasserrückhaltevermögen zeigt den, nach Abpressen von Fällungskieselsäuresuspension und Auswaschen auf Elektrolytarmut, enhaltenen Wasseranteil im Pressenteig auf. Zur Bestimmung werden 250 g Pressenteig bis zur Gewichtskonstanz bei 105°C getrocknet.

Berechnung:

$$100 - \frac{\text{Auswaage} \times 100}{\text{Einwaage}} = \% \text{ Wasserrückhaltevermögen}$$

Teilchengrößenverteilung
gemäß Coulter Counter/100/$\mu$m Kap.

Die Messung erfolgt mit dem Coulter Counter-Modell TA II der Fa. Coulter Eletronies GmbH

Bestimmung des Cu-Abrieb in 10 %iger Glyzerindispersion

a) Herstellung der Glyzerindispersion

153 g Glyzerin (wasserfrei, reinst Ph Eur, BP USP, Dichte = 1,26 g/ml, Fa. Merck, Darmstadt), werden in einem Polyäthylenbecher (250 ml) eingewogen. Mit dem Spatel werden vorsichtig 17 g Fällungskieselsäure untergemischt. Die Mischung wird anschließend mit einem Flügelrührer (Durchmesser 5,2 cm) 12 Minuten lang bei 1.500 Upm homogenisiert.

b) Durchführung der Abriebmessung

Die Bestimmung der Abriebmessung erfolgt in dem Abriebtestgerät, welches gemäß den folgenden Druckschriften bekannt ist: (1) Pfrengle: Fette, Seifen, Anstrichmittel, 63 (5) (1961), Seiten 445 bis 451 "Abrasion und Reinigungskraft von Putzkörpern in Zahnpasten" (2) A. RENG, F. DANY; Parfümerie und Kosmetik 59 (2) (1978), Seiten 37 bis 45; "Anwendungstechnische Prüfung von Zahnpasten". Dazu werden die 6 Tröge des Testgerätes mit je 20 ml der homogenen Dispersion beschichtet. Der Abrieb, den sechs plangeschliffene Nylonbürsten an sechs plangeschliffenen Cu-Blechen (Elektrolyt-Kupfer) in fünf Stunden mit 50.000 Doppelhüben bewirken, wird durch Differenzwägung bestimmt. Bei der Berechnung der Abrasivität werden von den erhaltenen Werten Mittelwerte gebildet. Der Abrieb (Abrasivität) wird in mg Cu angegeben.

RDA-Abrieb

REA-Abrieb

Die RDA-Methode wird beschrieben in Journal of Dental Research, 55 (4), 563 (1976), und wird auch für den REA-Abriebtest angewandt.
Kratzer werden visuell mittels Mikroskop bestimmt.

Bestimmung der Viskosität

gemessen in 16 %iger Glyzerin/Wasser-Dispersion (Gemisch 1:1) mit Broockfield-Viskosimeter RTV, Spindel 10, mit Helipath bei 10 UpM.

1. Probeansätze

```
   48 g Kieselsäure
  126 g Glyzerin (etwa 87%, reinst Ph Eur.
                 BP, Dichte = 1,23 g/ml
                 Fa. Merck, Darmstadt)
  126 g Wasser dest.
 ─────────
  300 g 16 %ige Dispersion bezogen auf
        Kieselsäure
```

2. Durchführung

Die Abrasivkieselsäurenwurden von Hand in einem 400 ml-Becherglas (breite Form) in das Glyzerin/Wasser-Gemisch mit einem Glasstab eingerührt (1 Minute) und 24 Stunden stehen gelassen. Danach wird die Viskosität gemessen.

3. Messung

Die Viskositätsmessung wird im selben Becherglas mit dem Brookfield-Viskosimeter RVT, Spindel 10, mit Helipath bei 10 UpM durchgeführt.

4. Ausrechnung

Abgelesener Skalenwert × Faktor = Viskosität in mPas.

**Patentansprüche**

EP 0 272 380 B1

1. Verfahren zur Herstellung von reinigenden, polierenden und verdickenden Fällungskieselsäuren für Zahnpflegemittel, dadurch gekennzeichnet, daß man die in üblicher Weise auf dem Fällungswege erhaltenen Fällungskieselsäuren unterschiedlicher Teilchengrössen und Teilchendichte in der Suspensionsphase homogen miteinander vermischt und die Gemische in üblicher Weise durch Filtrieren, Waschen, Trocknen und Vermahlen aufarbeitet.

2. Fällungskieselsäure, gekennzeichnet durch die folgenden physikalisch-chemischen Kenndaten:

| | | |
|---|---|---|
| Trocknungsverlust (DIN 53 198) | % | 3 – 7 |
| Leitfähigkeit bei $25^{\circ}C$ (4 %ige Aufschlämmung) | $\mu S$ | 400 – 800 |
| pH-Wert ( 5 % nach DIN 53 200) | | 6 – 7 |
| BET-Oberfläche (DIN 66 131) | $m^2/g$ | 130 – 150 |
| Stampfdichte (DIN 53 194) | g/l | 100 – 150 |

| | | |
|---|---|---|
| Makroporenvolumen D > 30 nm (nach der Hg-Einpreßmethode) | ml/g | 3,2 – 4,0 |
| Ölaufnahme n. Gardner | ml/100 g | 180 – 200 |
| Wasserrückhaltevermögen | % | 76 – 79 |

| | | |
|---|---|---|
| Cu-Abrieb | mg | 5 – 14 |
| RDA-Abrieb | | 35 – 100 |
| REA-Abrieb | | 40 – 90 |
| Kratzer | | wenig – sehr wenige |
| Viskosität (in 16 %iger Glyzerin-Wasser-Dispersion 1:1 Spindel D mit Helipath) | mPas | 5000 – 10 000 |
| Fe-Gehalt | ppm | 240 – 280 |

3. Verfahren zur Herstellung der Fällungskieselsäure nach Anspruch 2, die die folgenden physikalisch-chemischen Kenndaten aufweist:

13

| | | |
|---|---|---|
| Trocknungsverlust (DIN 53 198) | % | 3 - 7 |
| Leitfähigkeit bei 25$^{o}$C (4 %ige Aufschlämmung) | /uS | 400 - 800 |
| pH-Wert ( 5 % nach DIN 53 200) | | 6 - 7 |
| BET-Oberfläche (DIN 66 131) | m$^2$/g | 130 - 150 |
| Stampfdichte (DIN 53 194) | g/l | 100 - 150 |
| Makroporenvolumen D >30 nm (nach der Hg-Einpreßmethode) | ml/g | 3,2 - 4,0 |
| Ölaufnahme n. Gardner | ml/100 g | 180 - 200 |
| Wasserrückhaltevermögen | % | 76 - 79 |

| | | |
|---|---|---|
| Cu-Abrieb | mg | 5 - 14 |
| RDA-Abrieb | | 35 - 100 |
| REA-Abrieb | | 40 - 90 |
| Kratzer | | wenige - sehr wenige |
| Viskosität ( in 16 %iger Glyzerin- Wasser-Dispersion 1:1 Spindel D mit Helipath) | mPas | 5000 - 10 000 |
| Fe-Gehalt | ppm | 240 bis 280 |

dadurch gekennzeichnet, daß man zum einen eine Verdickungsfällungskieselsäuresuspension durch Umsetzung einer Alkalisilikatlösung mit einer Säure unter Vermeidung einer Gelbildung herstellt, wobei zur Fällung der Kieselsäure in eine vorgelegte Alkalisilikatlösung mit einer Konzentration von etwa 5 bis 25 g $SiO_2$ je Liter Lösung die Säure und die Alkalisilikatlösung mit bestimmten Lösungskonzentrationen und bestimmten Zulaufgeschwindigkeiten unter Aufrechterhaltung einer Fälltemperatur im Reaktionsmedium zwischen 80 und 90°C derartig eingespeist werden, daß die Viskosität des Reaktionsmediums in einem Zeitraum von mindestens 30 % der gesamten Fälldauer gleichmäßig niedrig und der pH-Wert zwischen 10 und 12 gehalten wird, und die Zugabe der Reaktionsteilnehmer beendet wird, bevor die Viskosität nach Durchlaufen eines Maximums auf einen weniger als 100 % über der Anfangsviskosität liegenden Wert gesunken ist, und der pH-Wert mit Schwefelsäure unter 7 eingestellt wird, zum anderen eine Abrasivfällungskieselsäuresuspension herstellt, indem man eine Originalfällungskieselsäuresuspension, die durch Fällung der Kieselsäure aus einer vorgelegten Alkalisilikatlösung mit einer Konzentration von etwa 5 - 25 g $SiO_2$ je Liter Lösung mit einer Säure und Alkalimetallsilikatlösung mit bestimmten Lösungskonzentrationen und bestimmten Zulaufgeschwindigkeiten unter Aufrechterhaltung einer Fälltemperatur im Reaktionsmedium zwischen 80 und 90°C , wobei gegebenenfalls der Behälterinhalt während der gesamten Fälldauer intensiv geschert wird, derart hergestellt wird, daß die Viskosität des Reaktionsmediums in einem Zeitraum von mindestens 30 % der gesamten Fälldauer gleichmäßig niedrig und der pH-Wert zwischen 10 und 12 gehalten wird, und die Zugabe der Reaktionsteilnehmer beendet wird, bevor die Viskosität nach Durchlaufen eines Maximums auf einen weniger als 100 % über der Anfangsviskosität liegenden Wert gesunken ist, den pH-Wert mit Schwefelsäure auf 7 bis 9 einstellt, mit heißem Wasser auf einen Fällungskieselsäuregehalt von 10 - 30 g/l und Natriumsulfatgehalt von 6 - 20 g $Na_2SO_4$/l verdünnt, auf 80 - 90°C erwärmt, und unter Konstanthalten dieses pH-Wertes durch gleichzeitigen Zulauf von Alkalimetallsilikatlösung, Schwefelsäure und gegebenenfalls heißem Wasser über eine Fälldauer von 15 bis 180 Minuten eine Fällungskieselsäureendkonzentration von 40 bis 80 g/l einstellt, die Suspension mit Schwefelsäure auf einen pH-Wert unter 7 ansäuert, beide Fällungskieselsäuresuspensionen miteinander vermischt, die Fällungskieselsäure abfiltriert wäscht, sprühtrocknet und anschliessend mit der Luftstrahlmühle vermahlt.

**4.** Fällungskieselsäure, gekennzeichnet durch die folgenden physikalisch-chemischen Kenndaten:

```
Trocknungsverlust (DIN 53 198)          %           3  -    7
Leitfähigkeit bei 25°C                 /uS        250  -  400
( 4 %ige Aufschlämmung)

pH-Wert ( 5 % nach DIN 53 200)                      6  -    7

BET-Oberfläche (DIN 66 131)           m²/g        220  -  250
Stampfdichte (DIN 53 194)             g/l          80  -  120

Makroporenvolumen D > 30 nm           ml/g        3,2  -  4,0
(nach der Hg-Einpreßmethode)

Ölaufnahme n. Gardner                 ml/100 g  200  -  220

Wasserrückhaltevermögen               %           77  -   78

Cu-Abrieb                             mg           8  -   12

RDA-Abrieb                                        60  -   80

REA-Abrieb                                        60  -   90

Kratzer                                           wenige

Viskosität                            mPas      14000- 18000
(in 16 %iger Glyzerin-
Wasser-Dispersion 1:1
Spindel D mit Helipath)

Fe-Gehalt                             ppm         70  -   90
```

**5.** Verfahren zur Herstellung der Fällungskieselsäure nach Anspruch 4, die die folgenden physikalisch-chemischen Kenndaten aufweist:

| | | | | |
|---|---|---|---|---|
| Trocknungsverlust (DIN 53 198) | % | 3 | – | 7 |
| Leitfähigkeit bei 25°C (4 %ige Aufschlämmung) | $\mu S$ | 250 | – | 400 |
| pH-Wert ( 5 % nach DIN 53 200) | | 6 | – | 7 |
| BET-Oberfläche (DIN 66 131) | $m^2/g$ | 220 | – | 250 |
| Stampfdichte (DIN 53 194) | g/l | 80 | – | 120 |
| Makroporenvolumen D > 30 nm (nach der Hg-Einpreßmethode) | ml/g | 3,2 | – | 4,0 |
| Ölaufnahme n. Gardner | ml/100g | 200 | – | 220 |
| Wasserrückhaltevermögen | % | 77 | – | 78 |
| Cu-Abrieb | mg | 8 | – | 12 |
| RDA-Abrieb | | 60 | – | 80 |
| REA-Abrieb | | 60 | – | 90 |
| Kratzer | | wenige | | |
| Viskosität (in 16 %iger Glyzerin-Wasser-Dispersion 1:1 Spindel D mit Helipath) | mPas | 14000 | – | 18000 |
| Fe-Gehalt | ppm | 70 | – | 90 |

dadurch gekennzeichnet, daß man zum einen eine Abrasivfällungskieselsäuresuspension herstellt, indem man eine Originalfällungskieselsäuresuspension, die durch Fällung der Kieselsäure aus einer vorgelegten Alkalisilikatlösung mit einer Konzentration von etwa 5 - 25 g $SiO_2$ je Liter Lösung mit einer Säure und Alkalimetallsilikatlösung mit bestimmten Lösungskonzentrationen und bestimmten Zulaufgeschwindigkeiten unter Aufrechterhaltung einer Fälltemperatur im Reaktionsmedium zwischen 80 und 90°C derart hergestellt wird, daß die Viskosität des Reaktionsmedium in einem Zeitraum von mindestens 30 % der gesamten Fälldauer gleichmäßig niedrig und der pH-Wert zwischen 10 und 12 gehalten wird, und die Zugabe der Reaktionsteilnehmer beendet wird, bevor die Viskosität nach Durchlaufen eines Maximums auf einen weniger als 100 % über der Anfangsviskosität liegenden Wert gesunken ist, den pH-Wert mit Schwefelsäure auf 7 bis 9 einstellt, mit heißem Wasser auf einen Fällungskieselsäuregehalt von 10 - 30 g/l und Natriumsulfatgehalt von 6 - 20 g $Na_2SO_4$/l verdünnt, auf 80 - 90°C erwärmt und unter Konstanthalten dieses pH-Wertes durch gleichzeitigen Zulauf von Alkalimetallsilikatlösung, Schwefelsäure und gegebenenfalls heißem Wasser über eine Fälldauer von 15 bis 180 Minuten eine Fällungskieselsäureendkonzentration von 40 bis 80 g/l einstellt, die Suspension mit Schwefelsäure auf einen pH-Wert unter 7 ansäuert, zum anderen eine weitere Fällungskieselsäuresuspension herstellt, indem man die Fällung im stark sauren pH-Bereich, vorzugsweise bei einem pH-Wert zwischen 1,5 und 2,5 durch gleichzeitige Zugabe der Wasserglas- und Säurelösungen zu einer Vorlage von in Wasser gelösten Stoffen, welche mit Eisen lösliche Komplexverbindungen eingehen, durchführt, beide Fällungskieselsäuresuspensionen miteinander vermischt, die Fällungskieselsäure abfiltriert, wäscht, sprühtrocknet und anschließend mit der Luftstrahlmühle vermahlt.

6. Verwendung der Fällungskieselsäure nach Anspruch 2 bzw. 4 als Abrasiv- bzw. Poliermittel in Zahnpasten.

**Claims**

1. A process for the production of cleaning, polishing and thickening precipitated silicas for tooth-care preparations, characterized in that the precipitated silicas varying in particle size and particle density obtained in the usual way by the precipitation method are homogeneously mixed with one another in the suspension phase and the resulting mixtures are worked up in the usual way by filtration, washing, drying and grinding.

2. A precipitated silica, **characterized** by the following physico-chemical data:

| | | |
|---|---|---|
| Drying loss (DIN 53 198) | % | 3 - 7 |
| Conductivity at 25°C (4% suspension) | $\mu$s | 400 - 800 |
| pH value (5% according to DIN 53 200) | | 6 - 7 |
| BET surface (DIN 66 131) | $m^2/g$ | 130 - 150 |
| Compacted bulk density (DIN 53 194) | g/l | 100 - 150 |
| Macropore volume D > 30 nm (Hg injection method) | ml/g | 3.2 - 4.0 |
| Gardner oil absorption | ml/100 g | 180 - 200 |
| Water retention capacity | % | 76 - 79 |
| Cu abrasion | mg | 5 - 14 |
| RDA abrasion | | 35 - 100 |
| REA abrasion | | 40 - 90 |
| Scratches | | few-very few |
| Viscosity (in 16% glycerol/water 1:1 dispersion, spindle D with Helipath) | mPas | 5,000-10,000 |
| Fe content | ppm | 240 - 280 |

3. A process for the production of the precipitated silica claimed in claim 2 having the following physico-chemical characteristic data:

| | | |
|---|---|---|
| Drying loss (DIN 53 198) | % | 3 - 7 |
| Conductivity at 25°C (4% suspension) | $\mu$s | 400 - 800 |
| pH value (5% according to DIN 53 200) | | 6 - 7 |
| BET surface (DIN 66 131) | $m^2/g$ | 130 - 150 |
| Compacted bulk density (DIN 53 194) | g/l | 100 - 150 |
| Macropore volume D > 30 nm (Hg injection method) | ml/g | 3.2 - 4.0 |
| Gardner oil absorption | ml/100 g | 180-200 |
| Water retention capacity | % | 76 - 79 |
| Cu abrasion | mg | 5 - 14 |
| RDA abrasion | | 35 - 100 |
| REA abrasion | | 40 - 90 |
| Scratches | | few-very few |
| Viscosity (in 16% glycerol/water 1:1 dispersion, spindle D with Helipath) | mPas | 5,000-10,000 |
| Fe content | ppm | 240 - 280, |

**characterized in that,** on the one hand, a thickening precipitated silica suspension is prepared by reaction of an alkali metal silicate solution with an acid avoiding gel formation, the acid and the alkali metal silicate solution - to precipitate the silica - being fed with certain solution concentrations and at certain rates into an alkali metal silicate solution having a concentration of about 5 to 25 g $SiO_2$ per litre solution at a precipitation temperature in the reaction medium kept between 80 and 90°C so that the viscosity of the reaction medium is kept uniformly low and the pH value is kept in the range from 10 to 12 over a period of at least 30% of the total precipitation time, and the addition of the reactants being terminated before the viscosity, after passing through a maximum, has fallen to a value less than 100% above the initial viscosity and the pH value being adjusted to below 7 with sulfuric acid; and in that, on the other hand, an abrasive precipitated silica suspension is prepared by diluting an original precipitated silica suspension - prepared by precipitation of the silica from an alkali metal silicate solution having a concentration of about 5 to 25 g $SiO_2$ per litre solution with an acid and an alkali metal silicate solution introduced with certain solution concentrations and at certain rates, a precipitation temperature of 80 to 90°C being maintained in the reaction medium and the contents of the vessel being intensively sheared throughout the precipitation process, so that the viscosity of the reaction medium is kept uniformly low and the pH value is kept in the range from 10 to 12 over a period of at least 30% of the total precipitation time, and the addition of the reactants being terminated before the viscosity, after passing through a maximum, has fallen to a value less than 100% above the initial viscosity and the pH value being adjusted to 7 - 9 with sulfuric acid - with hot water to a precipitated silica content of 10 - 30 g/l and a sodium sulfate content of 6 to 20 g $Na_2SO_4$/l, heating the suspension to 80 - 90°C and establishing a final precipitated silica concentration of 40 to 80 g/l by simultaneous introduction of alkali metal silicate solution, sulfuric acid and optionally hot water over a precipitation period of 15 to 180 minutes, during which the adjusted pH value is kept constant, subsequently acidifying the suspension with sulfuric acid to a pH value below 7, mixing both the precipitated silica suspensions with one another, filtering off, washing and spray-drying the precipitated silica and then grinding it in an air jet mill.

4. A precipitated silica, **characterized** by the following physico-chemical data:

| Drying loss (DIN 53 198) | % | 3 - 7 |
|---|---|---|
| Conductivity at 25°C (4% suspension) | µs | 250 - 400 |
| pH value (5% according to DIN 53 200) | | 6 - 7 |
| BET surface (DIN 66 131) | $m^2$/g | 220 - 250 |
| Compacted bulk density (DIN 53 194) | g/l | 80 - 120 |
| Macropore volume D > 30 nm (Hg injection method) | ml/g | 3.2 - 4.0 |
| Gardner oil absorption | ml/100 g | 200 - 220 |
| Water retention capacity | % | 77 - 78 |
| Cu abrasion | mg | 8 - 12 |
| RDA abrasion | | 60 - 80 |
| REA abrasion | | 60 - 90 |
| Scratches | | few |
| Viscosity (in 16% glycerol/water 1:1 dispersion, spindle D with Helipath) | mPas | 14000-18000 |
| Fe content | ppm | 70 - 90. |

5. A process for the production of the precipitated silica claimed in claim 4 having the following physico-chemical data:

| Drying loss (DIN 53 198) | % | 3 - 7 |
|---|---|---|
| Conductivity at 25°C (4% suspension) | µs | 250 - 400 |
| pH value (5% according to DIN 53 200) | | 6 - 7 |
| BET surface (DIN 66 131) | $m^2$/g | 220 - 250 |
| Compacted bulk density (DIN 53 194) | g/l | 80 - 120 |
| Macropore volume D > 30 nm (Hg injection method) | ml/g | 3.2 - 4.0 |
| Gardner oil absorption | ml/100 g | 200 - 220 |
| Water retention capacity | % | 77 - 78 |
| Cu abrasion | mg | 8 - 12 |
| RDA abrasion | | 60 - 80 |
| REA abrasion | | 60 - 90 |
| Scratches | | few |
| Viscosity (in 16% glycerol/water 1:1 dispersion, spindle D with Helipath) | mPas | 14000-18000 |
| Fe content | ppm | 70 - 90, |

**characterized in that,** on the one hand, an abrasive precipitated silica suspension is prepared by diluting an original precipitated silica suspension - prepared by precipitation of the silica from an alkali metal silicate solution having a concentration of about 5 to 25 g $SiO_2$ per litre solution with an acid and an alkali metal silicate solution introduced with certain solution concentrations and at certain rates, a precipitation temperature of 80 to 90°C being maintained in the reaction medium, so that the viscosity of the reaction medium is kept uniformly low and the pH value is kept in the range from 10 to 12 over a period of at least 30% of the total precipitation time, and the addition of the reactants being terminated before the viscosity, after passing through a maximum, has fallen to a value less than 100% above the initial viscosity and the pH value being adjusted to 7 - 9 with sulfuric acid - with hot water to a precipitated silica content of 10 - 30 g/l and a sodium sulfate content of 6 to 20 g $Na_2SO_4$/l, heating the suspension to 80 - 90°C and establishing a final precipitated silica concentration of 40 to 80 g/l by

simultaneous introduction of alkali metal silicate solution, sulfuric acid and optionally hot water over a precipitation period of 15 to 180 minutes, during which the adjusted pH value is kept constant, and subsequently acidifying the suspension with sulfuric acid to a pH value below 7; and in that, on the other hand, another precipitated silica suspension is prepared by carrying out the precipitation in the strongly acidic pH range, preferably at a pH value of 1.5 to 2.5 by simultaneous addition of the waterglass and acid solutions to a solution in water of substances which form soluble complex compounds with iron, mixing both the precipitated silica suspensions with one another, filtering off, washing and spray-drying the precipitated silica and then grinding it in an air jet mill.

6. The use of the precipitated silica claimed in claim 2 or 4 as an abrasive or polish in toothpastes.

**Revendications**

1. Procédé de préparation de silices précipitées épaississantes, polissantes et nettoyantes pour des dentifrices, caractérisé en ce qu'on mélange de manière homogène des silices obtenues par précipitation de granulométries et de densités de particules différentes en phase de suspension et qu'on traite les mélanges de manière habituelle par filtration, lavage, séchage et broyage.

2. Silice précipitée, caractérisée par les grandeurs physico-chimiques suivantes :

| | | |
|---|---|---|
| Perte au séchage (DIN 53198) | % | 3 - 7 |
| Conductibilité à 25°C (suspension à 4%) | µs | 400 - 800 |
| pH (à 5 % selon DIN 53200) | | 6 - 7 |
| Surface BET (DIN 66131) | $m^2/g$ | 130 - 150 |
| Densité après damage (DIN 53194) | g/l | 100 - 150 |
| Volume des macropores D > 30 nm (selon la méthode de pénétration de Hg) | ml/g | 3,2 - 4,0 |
| Prise d'huile selon Gardner | ml/100 g | 180 - 200 |
| Pouvoir de rétention d'eau | % | 76 - 79 |
| Abrasion-Cu | mg | 5 - 14 |
| Abrasion-RDA | | 35 - 100 |
| Abrasion REA | | 40 - 90 |
| Rayure | | peu -très peu |
| Viscosité (dans une dispersion à 16 % glycérine-eau 1:1 Tige D avec Helipath) | mPas | 5000 - 10000 |
| Teneur en Fe | ppm | 240 - 280 |

3. Procédé de préparation de silice précipitée selon la revendication 2 qui présente les valeurs physico-chimiques suivantes :

| | | |
|---|---|---|
| Perte au séchage (DIN 53198) | % | 3 - 7 |
| Conductibilité à 25°C (suspension à 4%) | µs | 400 - 800 |
| pH (à 5 % selon DIN 53200) | | 6 - 7 |
| Surface BET (DIN 66131) | $m^2/g$ | 130 - 150 |
| Densité après damage (DIN 53194) | g/l | 100 - 150 |
| Volume des macropores D > 30 nm (selon la méthode de pénétration de Hg) | ml/g | 3,2 - 4,0 |
| Prise d'huile selon Gardner | ml/100 g | 180 - 200 |
| Pouvoir de rétention d'eau | % | 76 - 79 |
| Abrasion-Cu | mg | 5 - 14 |
| Abrasion-RDA | | 35 - 100 |
| Abrasion REA | | 40 - 90 |
| Rayure | | peu -très peu |
| Viscosité (dans une dispersion à 16 % glycérine-eau 1:1 Tige D avec Helipath) | mPas | 5000 - 10000 |
| Teneur en Fe | ppm | 240 - 280 |

procédé caractérisé en ce qu'on réalise d'abord une suspension de silice précipitée épaississante par réaction d'une solution de silicate alcalin avec un acide en évitant la formation d'un gel, en alimentant pour précipiter la silice dans une solution de silicate alcalin déjà présente avec une concentration d'environ 5 à 25 g de SiO$_2$ par litre de solution, la solution de silicate alcalin et l'acide avec des concentrations déterminées de solution et des vitesses déterminées d'addition en maintenant une température de précipitation dans le milieu réactionnel comprise entre 80 et 90°C, de telle sorte que la viscosité du milieu réactionnel, dans un intervalle d'au moins 30 % de la durée totale de précipitation reste maintenue à une faible valeur et que le pH reste maintenu entre 10 et 12, et qu'on termine l'addition des partenaires de la réaction avant que la viscosité, après passage par un maximum, redescende à moins de 100 % au-dessus de la viscosité de départ et qu'on règle le pH avec de l'acide sulfurique en-dessous de 7, d'autre part on prépare une suspension de silice précipitée abrasive en préparant une suspension de silice précipitée originelle qu'on obtient par précipitation de silice à partir d'une solution de silicate alcalin présente ayant une concentration d'environ 5-25 g de SiO$_2$ par litre avec un acide et une solution de silicate de métal alcalin à des concentrations de solution définies et des vitesses d'addition définies en maintenant une température de précipitation dans le milieu réactionnel entre 80 et 90°C, en cisaillant intensément le cas échéant le contenu du récipient pendant toute la durée de la précipitation, de telle sorte que la viscosité du milieu réactionnel reste pendant un intervalle de temps d'au moins 30 % de toute la durée de précipitation régulièrement faible et que le pH soit maintenu entre 10 et 12, et on arrête l'addition des partenaires de la réaction, avant que la viscosité, après passage d'un maximum soit retombée à moins de 100 % au-dessus de la viscosité de départ, on règle le pH avec de l'acide sulfurique entre 7 et 9, on dilue avec de l'eau très chaude à une teneur en silice précipitée de 10-30 g/l et une teneur en sulfate de sodium de 6-20 g de Na$_2$SO$_4$/l, on chauffe à 80-90°C et on maintient constant ce pH par addition simultanée de solution de silicate de métal alcalin, acide sulfurique et le cas échéant eau très chaude pendant une durée de précipitation de 15 à 180 minutes, on ajuste une concentration finale en silice précipitée entre 40 et 80 g/l, on acidifie la suspension avec de l'acide sulfurique à un pH inférieur à 7, on mélange les deux suspensions de silice précipitée, on filtre la silice précipitée, on lave, atomise et ensuite on broie dans un broyeur à jet d'air.

4. Silice précipitée caractérisée par les données physico-chimiques suivantes :

| | | |
|---|---|---|
| Perte au séchage (DIN 53198) | % | 3 - 7 |
| Conductibilité à 25°C (suspension à 4 %) | µs | 250 - 400 |
| pH (à 5 % selon DIN 53200) | | 6 - 7 |
| Surface BET (DIN 66131) | m$^2$/g | 220 - 250 |
| Densité après damage (DIN 53194) | g/l | 80 - 120 |
| Volume des macropores D > 30 nm (selon la méthode de pénétration de Hg) | ml/g | 3,2 - 4,0 |
| Prise d'huile selon Gardner | ml/100 g | 200 - 220 |
| Pouvoir de rétention d'eau | % | 77 - 78 |
| Abrasion-Cu | mg | 8 - 12 |
| Abrasion-RDA | | 60 - 80 |
| Abrasion REA | | 60 - 90 |
| Rayure | | peu |
| Viscosité (dans une dispersion à 16 % glycérine-eau 1:1 Tige D avec Helipath) | mPas | 14000 - 18000 |
| Teneur en Fe | ppm | 70 - 90 |

5. Procédé de préparation de silice précipitée selon la revendication 4 qui présente les caractéristiques physico-chimiques suivantes :

| | | |
|---|---|---|
| Perte au séchage (DIN 53198) | % | 3 - 7 |
| Conductibilité à 25°C (suspension à 4 %) | $\mu s$ | 250 - 400 |
| pH (à 5 % selon DIN 53200) | | 6 - 7 |
| Surface BET (DIN 66131) | $m^2/g$ | 220 - 250 |
| Densité après damage (DIN 53194) | g/l | 80 - 120 |
| Volume des macropores D > 30 nm (selon la méthode de pénétration de Hg) | ml/g | 3,2 - 4,0 |
| Prise d'huile selon Gardner | ml/100 g | 200 - 220 |
| Pouvoir de rétention d'eau | % | 77 - 78 |
| Abrasion-Cu | mg | 8 - 12 |
| Abrasion-RDA | | 60 - 80 |
| Abrasion REA | | 60 - 90 |
| Rayure | | peu |
| Viscosité (dans une dispersion à 16 % glycérine-eau 1:1 Tige D avec Helipath) | mPas | 14000 - 18000 |
| Teneur en Fe | ppm | 70 - 90 |

procédé caractérisé en ce qu'on prépare une suspension de silice précipitée abrasive en préparant une suspension de silice précipitée originelle qu'on obtient par précipitation de silice à partir d'une solution de silicate alcalin ayant une concentration d'environ 5-25 g de $SiO_2$ par litre avec un acide et une solution de silicate de métal alcalin à des concentrations de solution définies et des vitesses d'addition définies, en maintenant une température de précipitation dans le milieu réactionnel entre 80 et 90°C de façon que la viscosité du milieu réactionnel reste pendant un intervalle de temps d'au moins 30 % de toute la durée de précipitation régulièrement faible et que le pH se maintienne entre 10 et 12, et on arrête l'addition des partenaires de la réaction, avant que la viscosité, après passage d'un maximum soit retombée à moins de 100 % au-dessus de la viscosité de départ, on règle le pH avec de l'acide sulfurique entre 7 et 9, on dilue avec de l'eau très chaude à une teneur en silice précipitée de 10-30 g/l et une teneur en sulfate de sodium de 6-20 g de $Na_2SO_4/l$, on chauffe à 80-90°C et en maintenant constant ce pH par addition simultanée de solution de silicate de métal alcalin, acide sulfurique et le cas échéant eau très chaude pendant une durée de précipitation de 15 à 180 minutes, on ajuste une concentration finale en silice précipitée entre 40 et 80 g/l, on acidifie la suspension avec de l'acide sulfurique à un pH inférieur à 7, d'autre part on prépare une autre suspension de silice préparée par précipitation, en réalisant la précipitation dans un domaine de pH fortement acide, de préférence à un pH compris entre 1,5 et 2,5 par l'addition simultanée de solution de silicate et d'acide à une solution de substances dissoutes dans l'eau, qui forment des composés complexes du fer solubles, on mélange les deux suspensions de silice précipitées, on filtre la silice précipitée, on lave, on sèche par pulvérisation et ensuite on broie dans un broyeur à jet d'air.

**6.** Utilisation de silice précipitée selon la revendication 2 ou 4, comme agent abrasif ou polisseur dans les pâtes dentifrices.

Fig. 1

EP 0 272 380 B1

KORNANALYSE

METHODE:
COULTER COUNTER
100 μm-KAPILLARE

*Fig. 2*

SUSPENSIONSGEMISCH

TROCKENGEMISCH

RÜCKSTAND [GEW.%] →

100 — 90 — 80 — 70 — 60 — 50 — 40 — 30 — 20 — 10 — 0

TEILCHENGRÖSSE [μm] →

0,5 — 1,0 — 5,0 — 10 — 50 — 100

← DURCHGANG [GEW.%]

0 — 10 — 20 — 30 — 40 — 50 — 60 — 70 — 80 — 90 — 100

23